# EUROPÄISCHE PATENTANMELDUNG

(11) **EP 2 074 944 A1**
(43) Veröffentlichungstag der Anmeldung: **01.07.2009**
(21) Anmeldenummer: 08022134.4
(22) Anmeldetag: 19.12.2008
(51) Int. Cl.: A61B 5/103, A61B 5/113, G01S 17/88, A61B 5/08

(54) **Vorrichtung zur multidimensionalen Atemdetektion mit einer Time-of-Flight Kamera**

(30) Priorität: 20.12.2007 DE 102007061893; 15.07.2008 DE 102008033243
(71) Anmelder: Softgate GmbH, 91052 Erlangen (DE); Friedrich-Alexander-Universität Erlangen-Nürnberg, 91054 Erlangen (DE)
(72) Erfinder: Schaller, Christian, 91054 Erlangen (DE); Penne, Jochen, 06918 Elster / Elbe (DE); Schuhmann, Peter, 91058 Erlangen (DE); Nyga, Udo, 91056 Erlangen (DE)
(74) Vertreter: Negendanck, Matthias

(57) **Zusammenfassung**

Die Erfindung betrifft eine Erfassungseinheit zur Atemdetektion. Die Erfassung eines multidimensionalen Atemsignals eines Patienten wird dadurch gelöst, dass ein ausgesandtes und am Rumpf des Patienten reflektiertes Lichtsignal gemäß des Time-of-Flight-Prinzipes und unter Nutzung einer Auswertungseinheit verarbeitet wird. Damit stehen für eine gewisse Anzahl von Punkten auf dem Rumpf des Patienten Tiefendaten zur Verfügung, welche ein multidimensionales Atemsignal repräsentieren.

## Beschreibung

### Technisches Gebiet der Erfindung

Die Erfindung betrifft eine Erfassungseinheit zur Atemdetektion. Genauer gesagt liegt die Erfindung auf dem Gebiet von bildgebenden Verfahren in der Medizin. Die Erfindung trägt dazu bei, eine Verbesserung bei Problemen durch Patientenatmung mit diesen Verfahren zu erzielen.

### Hintergrund der Erfindung

Durch die klinisch belegte, direkte Korrelation der Bewegung von inneren Organen und der Atmung des Patienten ermöglicht die Detektion von Atmung beispielsweise auf dem Gebiet der Computertomographie (CT), der Magnetresonanztomographie (MRI) und der Positronen Emmisions Tomographie (PET) die Möglichkeit Artefakte, die durch die Atmung des Patienten entstehen zu reduzieren.

In der Strahlentherapie lassen sich dadurch Tumorbewegungen besser vorhersagen und somit Tumore gezielter bestrahlen.

Herkömmliche Systeme zur Detektion von Atmung messen ein eindimensionales Atemsignal zum einen direkt über Sensoren am Patienten, oder auch mit Hilfe optischer Sensoren (CCD). Die menschliche Atmung ist jedoch in ihrer Entstehung ein sehr komplexer Prozess, welcher von vielen Faktoren abhängt. Der menschliche Körper ist im Stande sowohl über Muskeln des Brustkorbes (Brustatmung), als auch über das Zwerchfell (Bauchatmung) zu atmen. Anteilige Kombinationen dieser beiden Möglichkeiten sind zudem auch möglich. Hieraus entsteht eine Vielzahl von verschiedenen Bewegungsmustern innerhalb des Körpers, bedingt durch die unterschiedlichen Muskeln, die bei der Erzeugung von Atmung beteiligt sind. Daher ist es nicht ausreichend, die Atmung nur an einem speziellen Punkt zu messen. Für eine exakte Rekonstruktion oder eine genaue Bestimmung der Bewegung eines Tumors ist dies nicht ausreichend und heutzutage auch technologisch noch nicht gelöst.

Für eine akkurate Information über atmungsbedingte anatomische Bewegungen, die auf tatsächliche Veränderungen innerhalb des Patienten schließen lässt, ist es somit notwendig ein multidimensionales Atemsignal zu erfassen, welches in der Lage ist Bewegungen, spezifisch für bestimmte Körperregionen zu repräsentieren.

Die Erfassung eines solchen multidimensionalen Atemsignals muss zudem in Echtzeit erfolgen, da während eines Atemzyklus ständig auf auftretende anatomische Veränderungen reagiert werden muss.

### Kurzdarstellung der Erfindung

Diese Aufgabe wird durch eine Erfassungseinheit zur Detektion von Atmung gelöst. Eine erfindungsgemäße Erfassungseinheit umfasst erstens eine Modulationseinheit zur Ansteuerung der Lichtquelle gemäß Modulationsinformationen derart, dass die Lichtquelle ein moduliertes Lichtsignal aussendet, und zweitens eine mit dem Sensor verbundene Auswertungseinheit zur Auswertung des Sensorsignals unter Heranziehung der Modulationsinformationen, um Tiefendaten zu ermitteln. Der Begriff "Licht" ist nicht auf das sichtbare elektromagnetische Spektrum beschränkt, sondern umfasst auch z.B. den UV- oder Infrarotbereich. Die Erfassungseinheit nutzt das sog. Time-of-Flight (ToF) Prinzip.

Es handelt sich hierbei um ein physikalisches Prinzip, welches auf der Aussendung modulierten Lichtes beruht, dass von einer Szene reflektiert wird. Aus der gemessenen Zeit, die das modulierte Licht vom Emitter über die Reflektion an der Oberfläche einer Szene zurück zum ToF Sensor benötigt, wird eine Matrix von Tiefenwerten der Szene berechnet. Die Matrix der Tiefenwerte kann beispielsweise Dimensionen im Bereich 140x170 Pixel haben.

Mit Hilfe einer Matrix von solchen Tiefenwerten der Oberfläche eines Patienten ist es möglich, den Oberkörper in verschiedene Regionen einzuteilen

Innerhalb dieser Regionen kann die Bewegung der Oberfläche und somit die Atmung an der jeweiligen Stelle mit Hilfe der kontinuierlichen Aktualisierung der Tiefenmatrix durch den ToF Sensor gemessen werden.

Diese Messung erfolgt über die Beobachtung der Distanz der jeweiligen Region zum ToF Sensor über die Zeit.

Daraus resultiert ein mehrdimensionales Atemsignal, welches die Bewegung an verschiedenen Positionen des Oberkörpers repräsentiert.

Dieses multidimensionale Atemsignal kann zur Unterstützung der Verarbeitung jeglicher zeitvariant akquirierter Daten von beliebigen bildgebenden Verfahren verwendet werden.

Das multidimensionale Atemsignal kann auch für die Unterstützung der Behandlung von Tumoren mit Hilfe von geeigneten therapeutischen Verfahren verwendet werden. Ein solches Verfahren ist beispielsweise das atmungsadaptive Ansteuern von Linearbeschleunigern in der Onkologie.

Die Modulationsinformationen können die Amplitudenform (Rechteck, Sinus, etc.) oder die Frequenz des ausgesendeten Lichtsignals betreffen. Die Modulierung (oder Kodierung) kann aber auch etwa durch einzelne Lichtimpulse (sog. "Bursts") erfolgen.

Die Modulationsinformationen können in einer Steuerungseinheit in der Erfassungseinheit erzeugt und der Modulations- und Auswertungseinheit übermittelt werden. Jedoch ist eine Kopplung von Modulations- und Auswertungseinheit nicht unbedingt erforderlich. Die Auswertungseinheit muss lediglich zur Auswertung der Modulationen, die von der Modulationseinheit dem ausgesendeten Licht aufgeprägt werden, ausgebildet sein. Dies kann auch durch die Auswahl der für den Aufbau der Auswertungseinheit verwendeten Komponenten und/oder durch eine einmalige Konfiguration bei der Herstellung der Erfassungseinheit oder der Auswertungseinheit erreicht werden.

Die Auswertungseinheit kann zur Berechnung der Tiefendaten aus der Laufzeit des empfangenen Lichtsignals ausgebildet sein. Aus der Laufzeit kann der zurückgelegte Weg und damit die Entfernung eines bestrahlten Punktes berechnet werden. Die Laufzeit des Lichts kann direkt oder indirekt gemessen werden. Andere Auswertungsstrategien, die nicht laufzeitorientiert sind, können jedoch auch zum Einsatz gelangen.

Die Auswertungseinheit kann ferner zur Erstellung einer Matrix von Tiefendaten für eine Anzahl von Bildpunkten ausgebildet sein, indem für jeden Bildpunkt der (absolute oder relative) Abstand eines bestrahlten Objektes von der Erfassungseinheit berechnet wird.

Bei weiteren Ausführungsformen einer erfindungsgemäßen Erfassungseinheit wird das Prinzip des "Time-of-Flight" z.B. mittels eines Photonenmischdetektors (PMD), bei der Auswertung herangezogen. Hierbei handelt es sich um ein inkohärentes Laufzeitmessungsverfahren. Die einem PMD-Videosensor nachgeschaltete Auswertungseinheit liefert pixelweise Tiefen- und Intensitätsinformationen. Mit einer typischen Frequenz von 15-50 Hertz kann eine Matrix von Intensitätsinformationen bzw. - werten (entsprechend etwa einem Grauwertbild) und eine Matrix von Distanzwerten erzeugt werden. Die Intensitätsinformation betreffen etwa die reflektierte Amplitude oder Gesamtenergie. Die Auswertungseinheit kann ausgebildet sein Bilddaten aus den Intensitätsinformationen zu erzeugen. Weitere Einheiten oder Vorrichtungen zur Akquisition von Bilddaten sind in diesem Fall nicht unbedingt erforderlich.

Die Auswertungseinheit kann die Matrix von Tiefendaten in eine oder mehrere Regionen partitionieren und für jede Region ein Signal, welches die spezifische Atembewegung repräsentiert, bereitstellen.

Bei einer erfindungsgemäßen Erfassungseinheit kann die Auswertungseinheit und/oder eine Anzeigeeinheit zur Anzeige der Auswertungsdaten integriert angeordnet sein. Derartige Erfassungseinheiten sind kompakt und erfordern keine weiteren Komponenten. Die Auswertungseinheit kann etwa als DSP ("Digital Signal Processor") oder FPGA ("Fiel Programmable Gate Array") vorliegen.

Alternativ hierzu kann die Auswertungseinheit und/oder Anzeigeeinheit abgesetzt angeordnet sein. In diesem Fall können Teile der Auswertungseinheit etwa auf einem separaten Rechner implementiert sein und die Anzeigeeinheit wird durch die Anzeigeeinrichtung realisiert.

Ein erfindungsgemäßes Verfahren zur Detektion eines mehrdimensionalen Atemsignals bei einem Patienten umfasst die folgenden Schritte:
- Ansteuern einer Lichtquelle der Erfassungseinheit durch eine Modulationseinheit gemäß Modulationsinformationen derart, dass die Lichtquelle ein moduliertes Lichtsignal aussendet.
- Erzeugen eines das (durch Reflektion vom Rumpf des Patienten) empfangene Licht repräsentierenden Sensorsignals in der Auswertungseinheit
- Auswerten des Sensorsignals unter Heranziehung der Modulationsinformationen, um Tiefendaten zu berechnen.
- Auswertung der Tiefendaten zur Berechnung eines mehrdimensionalen Atemsignals, welches die atembedingte Bewegung repräsentiert

### Kurze Beschreibung der Figuren

Nachfolgend wird die Erfindung anhand von Ausführungsbeispielen erläutert, die in den beigefügten Zeichnungen dargestellt sind. Es zeigt:

**Fig. 1** eine schematische Darstellung eines ersten Ausführungsbeispiels einer erfindungsgemäßen Erfassungseinheit.

**Fig. 2** eine schematische Darstellung eines zweiten Ausführungsbeispiels einer erfindungsgemäßen Erfassungseinheit.

**Fig. 3** eine schematische Darstellung eines dritten Ausführungsbeispiels einer erfindungsgemäßen Erfassungseinheit.

**Fig. 4** eine schematische Darstellung eines vierten Ausführungsbeispiels einer erfindungsgemäßen Erfassungseinheit.

**Fig. 5** eine schematische Darstellung eines vierten Ausführungsbeispiels einer erfindungsgemäßen Erfassungseinheit.

**Fig. 6** in Form eines Flussdiagramms eine verfahrensmäßige Ausgestaltung der Erfindung.

### Beschreibung bevorzugter Ausführungsbeispiele

In der nachfolgenden Beschreibung werden konkrete Ausführungsbeispiele der Erfindung beschrieben, um die Aspekte, Vorteile und Zweckmäßigkeiten der Erfindung weiter zu veranschaulichen. Dem Fachmann ist offenkundig, dass weitere Anwendungen der Erfindung möglich sind, die in fachmännischen Details von den hier geschilderten abweichen.

Funktionale Aspekte der Erfindung können in Form von Hardware, Firmware, Software oder einer Kombination hiervon implementiert werden. Die Funktion von in Soft- oder Firmware ausgeführten Aspekten ergibt sich mit der Ausführung auf einem entsprechend programmierten Prozessor, beispielsweise einem allgemein verwendbaren Prozessor oder einem für bestimmte Anwendungen optimierten Prozessor, z.B. einem ASIC ("Application Specific Integrated Circuit") oder einem programmierten DSP ("Digital Signal Processor"). Ein erfindungsgemäßes Verfahren kann durch die Ausführung eines in einem Speicher gespeicherten Programms auf einem Prozessor realisiert werden. Statt eines Programms können auch mehrere Programme vorliegen.

In der nachfolgenden Beschreibung bevorzugter Ausführungsbeispiele der Erfindung werden gleiche und gleich wirkende Teile mit denselben Bezugsziffern bezeichnet.

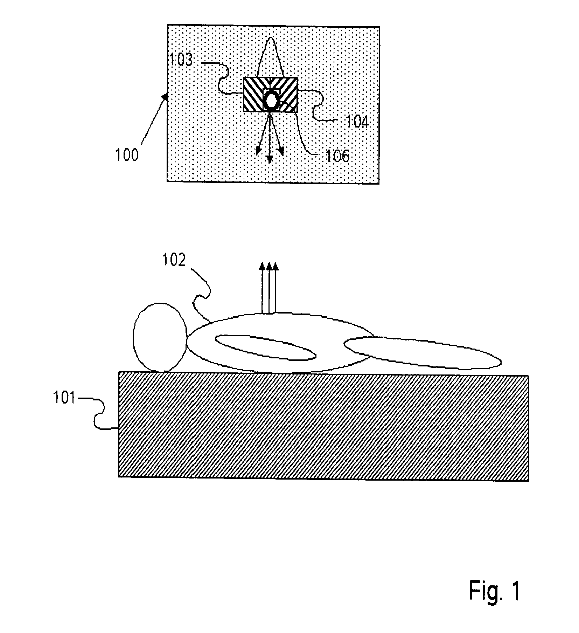

Fig. 1 zeigt in schematischer Form ein erstes Ausführungsbeispiel einer erfindungsgemäßen Erfassungseinheit **100** mit einer Lichtquelle und Modulationseinheit **103** und einer Auswertungseinheit **104.** Lichtquelle und Modulationseinheit sind aus Gründen der Klarheit der Darstellung Fig.1 in einer geschlossenen Einheit dargestellt. Die Erfassungseinheit **100** kann beispielsweise in einem Winkel von 0° zur Normale einer Vorrichtung **101** zur Lagerung des Patienten **102** angebracht werden. Auch andere Winkel oder Positionen sind denkbar. Die Auswertungseinheit **104** kann auf dem Prinzip des "Time-of-Flight" basieren und als Bestandteil beispielsweise eine Kamera des Typs PMD [vision] 19K der Firma PMD Technologies GmbH, D-57076 Siegen, beinhalten.

Das von der Lichtquelle **106** abgestrahlte Licht fällt beispielsweise auf einen Patienten **102** und wird von diesem in Abhängigkeit etwa von seiner Oberflächenbeschaffenheit bzw. Textur und seinem Reflektionsvermögen in Richtung einer geeignet montierten Auswertungseinheit **104** reflektiert.

Das reflektierte Licht wird in der Auswertungseinheit **104** von einem optischen in ein elektrisches Signal umgewandelt. Dies kann beispielsweise durch einen "Time-of-Flight"-basierten Sensor geschehen.

Das von der Lichteinheit **106** abgestrahlte Licht kann dabei entsprechend [0016] gemäß verschiedener Verfahren moduliert werden. Die Modulationsfrequenz kann etwa bei 20 Megahertz (Mhz) liegen, kann aber auch andere Werte annehmen.

Das reflektierte Licht weist immer doch die aufgeprägte Kodierung bzw. Modulierung auf. Die Auswertungseinheit **104** erhält zusätzlich die Modulationsinformationen von der Modulationseinheit **103** und ermittelt die Laufzeitverzögerung des reflektierten Lichtsignals (z.B. aus der Phasenverschiebung der Modulation des reflektierten Lichtsignals gegen die Modulationsinformationen).

Aus der ermittelten Laufzeit kann der Abstand des Patienten **102** von der Auswertungseinheit **104** (bzw. Lichtquelle in Modulationseinheit **103)** berechnet werden.

Die Auswertungseinheit **104** führt die Berechnung für eine Matrix von Bildpunkten durch, zum Beispiel 140x170 Pixel. Für jeden Bildpunkt wird die Laufzeit des auf diesen Punkt treffenden, reflektierten Lichts und somit der zurückgelegten Weg bestimmt. Aus dem Weg kann wiederum der Abstand eines bestrahlten Patienten **102** von der Auswertungseinheit **104** berechnet werden. Die Abstandsinformation für jeden Bildpunkt kann bei Kenntnis der intrinsischen Kameraparameter, die z.B. in einem Kalibrierschritt bestimmt werden können, in ein kartesisches (Welt-)Koordinatensystem überführt werden. Bezogen auf Fig.1 liegen dann metrische Tiefeninformationen für diejenigen Bildpunkte der Auswertungseinheit **104** vor, auf die von dem Patienten **102** reflektiertes Licht fällt.

Die Lichtquelle **106** strahlt Infrarot(IR)-Licht bei einer Wellenlänge von 870 nm aus. Die genaue Wellenlänge ist für die Funktionsweise der Erfindung jedoch ohne Belang. Es können ohne weiteres auch andere Wellenlängen oder Wellenlängenbereiche verwendet werden, bspw. im bereich von 500 nm-1000 nm.

Bei der oben beschriebenen Betriebsweise liefert die Erfassungseinheit **100** Tiefendaten mit einer Frequenz von etwa 15 Hz. Höhere oder niedrigere Erfassungsfrequenzen sind ebenfalls möglich. Bei dem in der Fig.1 gezeigten Ausführungsbeispiel ist die Auswertungseinheit **104** mit den anderen Komponenten der Erfassungseinheit **100** integriert. Andere Konfigurationen sind möglich und werden weiter unten vorgestellt. Die Auswertungseinheit **104** bzw. -elektronik kann etwa als DSP ("Digital Signal Processor") oder FGPA ("Field Programmmable Gate Array") vorliegen. Gleiches gilt auch für die Modulationseinheit **103.**

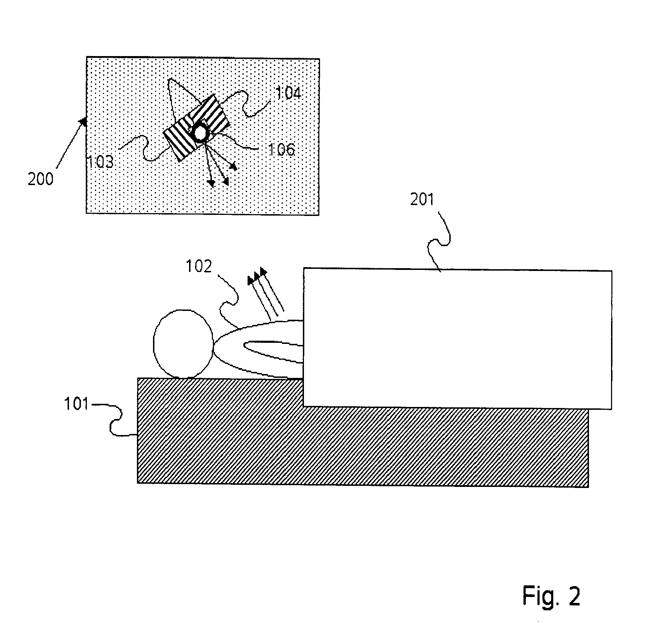

[0042] Fig.2 zeigt ein weiteres Ausführungsbeispiel einer erfindungsgemäßen Erfassungseinheit **200.** Dieses unterscheidet sich von dem in Fig.1 gezeigten dadurch, dass der auf einer Unterlage **101** liegende Patient **102** durch eine Abdeckvorrichtung **201** teilweise nicht direkt einsehbar ist. Die Verdeckung kann aus Sicht der Modulationseinheit **103** und Auswertungseinheit **104** eine nur partielle Sichtbarkeit erlauben. Teile von Modulationseinheit **103** und Auswertungseinheit **104** haben mindestens freie Sicht auf den Oberkörper des Patienten **102.**

[0043] Die Abdeckvorrichtung **201** kann durch einen CT-Scanner oder einen PET-Scanner realisiert sein. Andere Ausprägungen sind jedoch auch möglich und für die Anwendbarkeit der Erfindung ohne Belang.

Die Funktionsweise des erfindungsgemäßen Ausführungsbeispieles kann äquivalent zu den in [0036] bis [0043] beschriebenen Aspekten beschrieben werden.

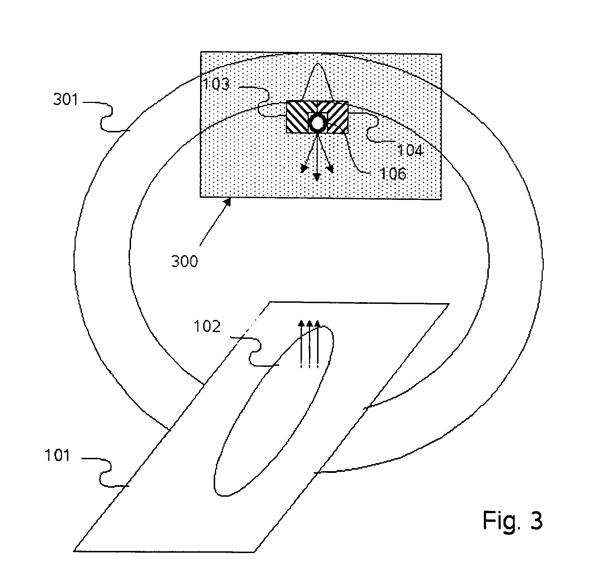

Fig.3 zeigt ein weiteres Ausführungsbeispiel eines erfindungsgemäßen Erfassungseinheit **300.** Gegenüber der in Fig.2 dargestellten Erfassungseinheit **200** sind hier Modulationseinheit **103** und Auswertungseinheit **104** innerhalb der Abdeckvorrichtung **301**angebracht.

Die Abdeckung des auf einer Unterlage **101** liegenden Patienten **102** kann dabei beliebig sein.

Von Teilen der Modulationseinheit **103** und Auswertungseinheit **104** kann mindestens der Oberkörper vom Hals bis zum Bauchnabel wahrgenommen werden.

Die Abdeckvorrichtung **301** kann durch einen CT-Scanner oder PET-Scanner realisiert werden. Andere Ausprägungen sind möglich und für die Funktionsweise der Erfindung ohne Belang.

Die Funktionsweise des erfindungsgemäßen Ausführungsbeispieles kann äquivalent zu den in [0036] bis [0043] beschriebenen Aspekten beschrieben werden.

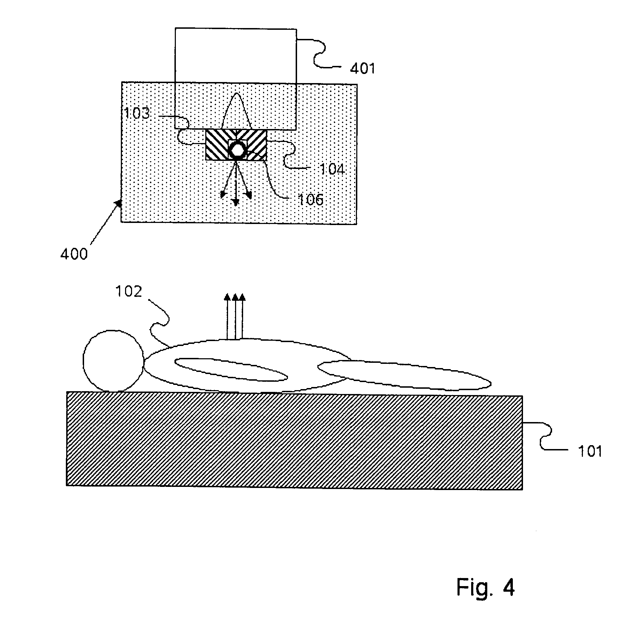

Fig.4 zeigt ein weiteres Ausführungsbeispiel eines erfindungsgemäßen Erfassungseinheit **400.** Gegenüber der in Fig.3 dargestellten Erfassungseinheit **400** sind hier Modulationseinheit **103** und Auswertungseinheit **104** an einer separaten, den auf einer Unterlage **101** gelagerten Patienten **102** nicht verdeckenden Konstruktion **402** angebracht und Teile von Modulationseinheit **103** und Auswertungseinheit **104** haben mindestens freie Sicht auf den Oberkörper des Patienten **102.**

Die Konstruktion **401** kann transliert und rotiert werden und kann beispielsweise an einem Linearbeschleuniger, wie er in der Tumortherapie eingesetzt wird, montiert sein. Andere Ausprägungen sind jedoch möglich und für die Funktionsweise der Erfindung ohne Belang.

Die Funktionsweise des erfindungsgemäßen Ausführungsbeispieles kann äquivalent zu den in [0036] bis [0043] beschriebenen Aspekten beschrieben werden.

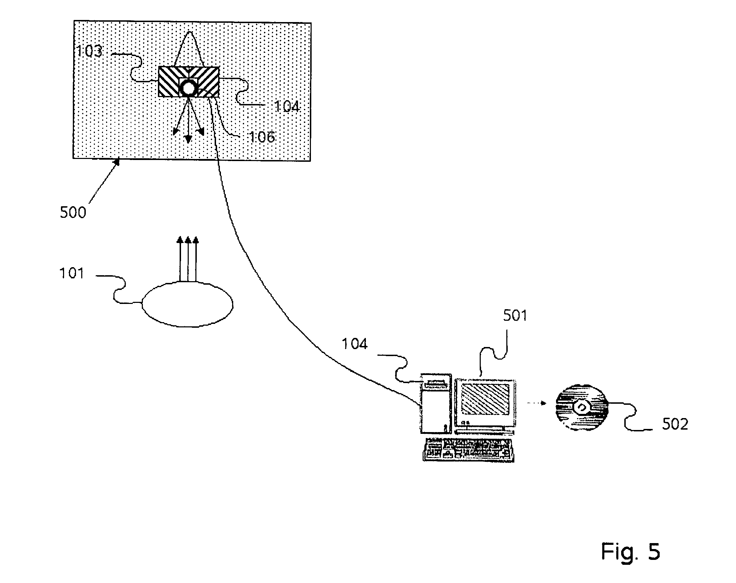

Fig.5 zeigt ein erfindungsgemäßes Ausführungsbeispiel einer Erfassungseinheit **500,** welches sich von den vorherigen Beispielen dadurch unterscheidet, dass Teile der Auswertungseinheit **104,** abgesetzt angeordnet, etwa in Form von Software auf einem handelsüblichen PC **104.** Gleiches kann auch für die Modulationseinheit **103** gelten. Die Auswertungs **104 -** und/oder Modulationseinheit **103** könnten auch nur auf einem PC oder einer programmierbaren Software vorhanden sein.

Als Anzeigeeinrichtung wird ein Monitor **501** des PCs verwendet. Zur Darstellung können 2D- oder 3D-Displaytechnologien verwendet werden.

Die Auswertungseinheit bzw. -elektronik **104** kann eine Komponente zur Ausgabe von Daten auf ein Speichermedium **502** umfassen.

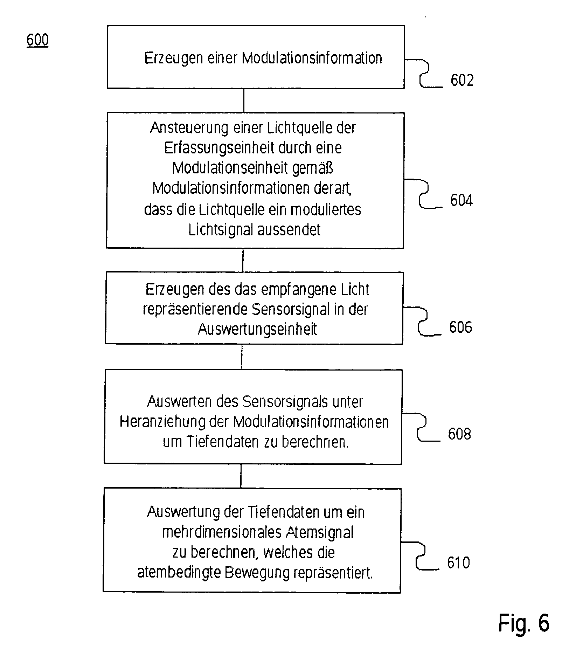

In Fig.6 sind für ein weiteres Ausführungsbeispiel die Schritte einer verfahrensmäßigen Ausgestaltung **600** der Erfindungs- wiedergegeben. Ein Verfahren zur Detektion eines mehrdimensionalen Atemsignals beginnt im Schritt **602** mit dem Erzeugen einer Modulationsinformation, die im Schritt **604** zur Ansteuerung einer Lichtquelle der Erfassungseinheit **(100,200,300,400,500)** durch eine Modulationseinheit **103** gemäß Modulationsinformationen derart genutzt wird, dass die Lichtquelle ein moduliertes Lichtsignal aussendet. In einem weiteren Schritt **606** wird ein das empfangene Licht repräsentierende Sensorsignal in der Auswertungseinheit **104** erzeugt. Anschließend werden in Schritt **608** durch das Auswerten des Sensorsignals unter Heranziehung der Modulationsinformationen Tiefendaten berechnet.
Schließlich wird in Schritt **610** durch die Auswertung der Tiefendaten ein mehrdimensionales Atemsignal berechnet, welches die atembedingte Bewegung repräsentiert.

Die Erfindung ermöglicht die Akquisition von Tiefendaten in Echtzeit. Die Bereitstellung des mehrdimensionalen Atemsignales kann ebenfalls in Echtzeit erfolgen. Die Erfindung ist für alle Anwendungsgebiete bei denen atmungsinduzierte Bewegungen auftreten anwendbar. Dies umfasst beispielsweise alle zeitvariant akquirierten medizinischen Bildinformationen.

Die hier dargestellten Ausführungsbeispiele stellen zweckmäßige Ausführungsformen der Erfindung dar. Im Rahmen eines erfindungsgemäßen Grundgedankens sind durch fachmännisches Handeln weitere Ausführungsformen denkbar, ohne dass der Geltungsbereich der Erfindung verlassen wird, der allein durch die beigefügten Ansprüche beschrieben werden soll.

## Patentansprüche

1. Erfassungseinheit **(100, 200, 300, 400, 500)** zur Detektion eines mehrdimensionalen Atemsignals mit
- einer Vorrichtung **(101)** zur Lagerung des Patienten **(102),**
- einer Lichtquelle **(103),**
- einem Sensor **(103),** um vom Rumpfbereich des Patienten reflektiertes Licht zu empfangen und ein das empfangene Licht repräsentierendes Sensorsignal zu erzeugen, **gekennzeichnet durch**
- eine Modulationseinheit **(103)** zur Ansteuerung der Lichtquelle **(106)** gemäß Modulationsinformationen derart, dass die Lichtquelle **(106)** ein moduliertes Lichtsignal auf den Rumpfbereich des Patienten **(102)** aussendet, und
- eine mit dem Sensor **(103)** verbundenen Auswertungseinheit **(104)** zur Auswertung des Sensorsignals unter Heranziehung der Modulationsinformationen, um Tiefendaten zu berechnen, die zur Berechnung des mehrdimensionalen Atemsignals verwendet werden.

2. Erfassungseinheit **(100, 200, 300, 400, 500)** nach Anspruch 1, **gekennzeichnet durch** eine Steuerungseinheit, die in der Modulationseinheit (103) integriert ist, zur Erzeugung der Modulationsinformationen.

3. Erfassungseinheit **(100, 200, 300, 400, 500)** nach Anspruch 1 oder 2, **dadurch gekennzeichnet, dass** die Auswertungseinheit **104**) zur Berechnung der Tiefendaten aus der Laufzeit des empfangenen Lichtsignals ausgebildet ist.

4. Erfassungseinheit **(100, 200, 300, 400, 500)** nach einem der vorhergehenden Ansprüche, **dadurch gekennzeichnet, dass** die Auswertungseinheit **(104)** zur Erstellung einer Matrix von Tiefendaten für eine Anzahl von Bildpunkten ausgebildet ist, indem für jeden Bildpunkt der Abstand eines bestrahlten Objektes von der Lichtquelle **(106)** berechnet wird.

5. Erfassungseinheit **(100, 200, 300, 400, 500)** nach einem der vorhergehenden Ansprüche, **dadurch gekennzeichnet, dass** die Auswertungseinheit **(104)** ausgebildet ist, um die Modulationsinformationen gemäß der Technologie der Photonenmischdetektoren bei der Auswertung heranzuziehen.

6. Erfassungseinheit **(100, 200, 300, 400, 500)** nach Anspruch 5, **dadurch gekennzeichnet, dass** die Auswertungseinheit ausgebildet ist, Bilddaten aus Intensitätsinformationen zu erzeugen, die gemäß der Technologie der Photonenmischdetektoren erzeugt werden.

7. Erfassungseinheit nach einem der vorhergehenden Ansprüchen, **dadurch gekennzeichnet, dass** die Auswertungseinheit **(104)** zur Auswertung der Tiefendaten ausgebildet ist.

8. Erfassungseinheit **(100, 200, 300, 400, 500)** nach Anspruch 7, **dadurch gekennzeichnet, dass** die Auswertungseinheit **(104)** zur Detektion eines mehrdimensionalen Atemsignals ausgebildet ist, basierend auf Bilddaten auf Intensitätsinformationen, die gemäß der Technologie der Photonenmischdetektoren erzeugt werden.

9. Erfassungseinheit **(100, 200, 300, 400, 500)** nach einem der vorhergehenden Ansprüche, **dadurch gekennzeichnet, dass** die Auswertungseinheit **(104)** und/oder eine Anzeigeeinheit **(501)** zur Anzeige der Auswertungsdaten integriert angeordnet sind.

10. Erfassungseinheit **(100, 200, 300, 400, 500)** nach einem der Ansprüche 1 bis 9, **dadurch gekennzeichnet, dass Auswertungseinheit (104) und/oder Anzeigeeinheit (501)** abgesetzt angeordnet sind.

11. Verfahren zur Akquisition von Tiefendaten zur Detektion eines mehrdimensionalen Atemsignals bei einem Patienten **(102)** mit einer Erfassungseinheit **(100,200,300,400,500),** mit den folgenden Schritten:
- Ansteuern einer Lichtquelle **(103)** der Erfassungseinheit **(100,200,300,400,500)** gemäß Modulationsinformationen derart, dass die Lichtquelle **(106)** ein moduliertes Lichtsignal aussendet,
- Aufnehmen des von der Lichtquelle **(106)** ausgesendeten und vom Rumpfbereich des Patienten **(102)** reflektierten Lichtes durch einen Sensor **(103)**
- Erzeugen eines das empfangene Licht repräsentierenden Sensorsignals,
- Auswerten des Sensorsignals unter Heranziehung der Modulationsinformation, um Tiefendaten zu berechnen und
- Auswerten der Tiefendaten zur Berechnung eines multidimensionalen Atemsignals.

12. Verfahren nach Anspruch 11, **gekennzeichnet durch** den Schritt des Erzeugens der Modulationsinformationen, der dem Ansteuern der Lichtquelle **(106)** der Erfassungseinheit **(100,200,300,400,500)** vorgelagert ist.

13. Verfahren nach Anspruch 11 oder 12, **dadurch gekennzeichnet, dass** die Tiefendaten aus der Laufzeit des empfangenen Lichtsignals berechnet werden.

14. Verwendung eines Photonenmischdetektors für eine Erfassungseinheit zur Detektion eines mehrdimensionalen Atemsignals.
